# EUROPEAN PATENT SPECIFICATION

(11) **EP 2 520 303 B1**
(45) Date of publication and mention of the grant of the patent: **18.05.2016**
(21) Application number: 12154279.9
(22) Date of filing: 07.02.2012
(51) Int. Cl.: A61K 31/706, A61K 36/81, A61P 17/12, A61K 9/00, A61K 9/06

(54) **ANTI-WART PHARMACEUTICAL COMPOSITION**
Pharmazeutische Zusammensetzung gegen Warzen
Composition pharmaceutique anti-verrues

(30) Priority: 04.05.2011 TW 100115591
(43) Date of publication of application: 07.11.2012
(73) Proprietor: G & E Herbal Biotechnology Co., Ltd., Tainan (TW)
(72) Inventor: KUO, Kou-Wha, Kaohsiung City (TW); SHEU, Hamm-Ming, Kaohsiung City (TW)
(74) Representative: Srinivasan, Ravi Chandran

(56) References cited:
- EP-A1- 0 020 029
- EP-A1- 1 508 334
- WO-A1-00/61153
- AU-B2- 540 812

## Description

This application claims priority of Taiwanese application no. 100115591, filed on May 4, 2011.

This invention relates to an anti-wart pharmaceutical composition containing a water-soluble extract from a plant of *Solanum* genus and a method for treating wart using the water-soluble extract.

Wart is a kind of dermatosis that occurs on the skin or mucous of human or animal subjects, and is a benign epidermal hyperplasia caused by infection of Human papilloma virus (HPVs). In general, based on the site where the wart forms, wart can be divided into cutaneous wart and mucosal wart. The epidermal cells of skin or mucosa affected by HPVs are subjected to benign proliferation and hyperkeratosis, thereby resulting in formation of papules or nodules with roughened surfaces on the skin or mucosa.

In clinical diagnosis, wart is divided into four types according to symptoms thereof: (1) common wart (*Verruca vulgaris*), scaly and roughened papule(s) or nodule(s) which usually occur on hands or fingers ; (2) flat wart (*Verruca plana juvenilis*), slightlyraisedandflat-topped papule (s) which usually occurs on faces, hands, and lower legs; (3) plantar wart (*Verruca plantaris*), a thick and hyperkeratotic papule which usually occurs at pressure points of soles and toes and causes painful feeling for a patient when the papule is pressed; and (4) genital wart (*Condylama acuminatum*)*,* a cauliflower-like lump formed on skin or mucosa of genitalia and anus, which is a highly infectious and sexually transmitted disease (STD).

Nowadays, warts are treated using the following remedies based on the type, size, number, and lesion site thereof:
(1) Topical therapy, e.g., podophyllin (Trade name: Wartec^{®}) and imiquimod (trade name: Aldara^{®}) for treating genital wart, 5-fluorouracil for treating genital wart and cutaneous wart, and bleomycin (Trade name: Bleocin^{®}) for treating plantar wart and warts formed on palm;
(2) Laser treatment, e. g. , pulsed dye laser and carbon dioxide laser, especially for treating plantar wart and warts formed on palm; and
(3) Cryotherapy using liquid nitrogen and suitable for treating most warts, less effective for warts formed on palm, sole, and toe.

Each of the aforesaid remedies is suitable only for a specific type of wart, rather than all types of warts. Moreover, in clinical diagnosis, there are inferior effect problem and side effect drawback for the aforesaid remedies. For example, topical therapy used for a long period of time will cause recurrence of warts and treating effect thereof is unsatisfactory. Laser treatment and cryotherapy will cause severe side effects, such as pain, ulceration, and scarring. Some patients, especially children, are likely to refuse treatment due to the unendurable severe side effects.

To overcome the aforesaid drawbacks, researches have focused on finding active components from traditional Chinese medicines or herbs to develop a drug that is effective for treating warts and that has no undesired side effects.

It is known that *Solanum incanum* L. (also known as *Solanum incanum* Ruiz. & Pair., *Solanum undatum, Solanum coagulans Forsskal* in Latin, and bitter apple in English) contains steroidal glycoalkaloid which exhibits anti-cancer activity. In addition, many plants of the *Solanum* genus are reported to contain steroidal glycoalkaloid, including, for example, *Solanum indicum, Solanum nigrum,* also known as Long Kui in Chinese and black nightshade in English, *Solanum capsicastrum* (known as false Jerusalem cherry in English, *Solanum xanthocarpum, Solanum melongena, Solanum coagulans, Solanum tuberosum, Solanum sodomeum*, *Solanum turburosum, Solanum aculeastrum, Solanum lycocarpum, Solanum khasianum, Solanum suaveolens, Solanum uporo, Solanum abutiloides, Solanum coccineum, Solanum unguiculatum, Solanum robustum, Solanum anguivi, Solanum platanifolium, Solanum mammosum,* etc. The common steroidal glycoalkaloids contained in plants of *Solanum* genus include solamargine and solasonine.

US 7,078,063 B2 (and EP 1 508 334 A1) issued to the inventors of this invention discloses a water soluble extract from a plant of *Solanum* genus, especially *Solanum incanum* L., and a process for preparing the same. The water soluble extract consists essentially of at least 60 wt% of solamargine and solasonine.

In the aforesaid US patent, the inventors found that the water soluble extract exhibits inhibition of growth of tumor/cancer cells, in particular liver tumor cells, lung cancer cells, and breast cancer cells. In this invention, the inventors unexpectedly found that the water soluble extract is effective in treating warts.

Therefore, this invention provides a water-soluble extract from a plant of *Solanum* genus for use in the treatment of wart on the skin or mucous of a human or animal subject, said water-soluble extract comprising solamargine and solasonine, and being obtainable by the steps (a) to (g) described below.

This invention relates to the use of the water-soluble extract in the manufacture of a medicament for treating wart,

r EP 0 020 029 A1 and AU-B-57853/80 disclose water-insoluble extracts of the genus *Solanum* (BEC-001 extract) for the treatment of warts.

The above and other objects, features and advantages of this invention will become apparent with reference to the following detailed description and the preferred embodiments taken in conjunction with the accompanying drawings, in which:
Fig. 1 shows photos illustrating that lesions (indicated by arrows) of test subject C are gradually ameliorated by treating the same with *a Solanum incanum* L. gel;
Fig. 2 shows photos illustrating that lesions (indicated by arrows) of test subject D are gradually ameliorated by treating the same with a *Solanum incanum* L. gel;
Fig. 3 shows photos illustrating that lesions (indicated by arrows) of test subject E are gradually ameliorated by treating the same with a *Solanum incanum* L. gel; and
Fig. 4 shows photos illustrating that lesions (indicated by arrows) of test subject F are gradually ameliorated by treating the same with a *Solanum incanum* L. gel.

It is to be understood that, if any prior art publication is referred to herein, such reference does not constitute an admission that the publication forms a part of the common general knowledge in the art, in Taiwan or any other country.

For the purpose of this specification, it will be clearly understood that the word "comprising" means "including but not limited to", and that the words "comprises," "contain" and variants thereof have a corresponding meaning.

The present invention provides a water-soluble extract from a plant of *Solanum* genus for use in the treatment of wart on the skin or mucous of a human or animal subject, which water-soluble extract is obtainable by the steps (a) to (g) as described below, and which water-soluble extract comprises solamargine and solasonine. Preferably, the water-soluble extract comprises at least 60 wt% of solamargine and solasonine, more preferably, 60 wt%-90 wt% of solamargine and solasonine.

The process for preparing the water-soluble extract has been disclosed in US 7,078,063 B2 and includes the steps of:
(a) subjecting a plant material of a plant of *Solanum* genus to an extraction treatment using an acidic aqueous solution with a pH value of 3~5, such that an aqueous solution is obtained;
(b) adjusting the pH value of the aqueous solution obtained in step (a) to pH 8∼10 with a base, such that a precipitate is formed;
(c) washing the precipitate formed in step (b) with water, followed by drying, such that a dried product is obtained;
(d) admixing the dried product obtained in step (c) with chloroform, followed by addition of a suitable amount of a 100% alcohol, such that a chloroform-alcohol mixture is formed;
(e)mixing the chloroform-alcohol mixture formed in step (d) with a water/alcohol solution having a predetermined water:alcohol ratio, such that a mixture containing a chloroform-based layer and a non-chloroform-based layer is obtained;
(f) removing the chloroform-based layer from the mixture obtained in step (e), followed by addition of a suitable amount of water; and
(g) obtaining a supernatant from the resultant mixture of step (f), followed by drying the supernatant, wherein the resultant dried product is able to be directly dissolved in water to forma yellowish clear and transparent aqueous solution.

Preferably, in step (a), the plant material of the plant of *Solanum* genus has been chopped in a preliminary treatment.

Preferably, in step (a), the plant material is at least one of the fruit, root, stem, and leaf of the plant of *Solanum* genus. In a preferred embodiment of this invention, the plant material used in step (a) is the fruit of the plant of *Solanum* genus.

The inventors found that certain factors might affect the content and proportion of solasonine and solamargine in the water-soluble extract obtained using the aforesaid process. These factors include the species of the plant of *Solanum* genus and the part/parts of the plant used in the extracting process, as well as the types of alcohol and base used. Therefore, a skilled artisan can prepare a desired water-soluble extract by selecting a suitable species of the plant of *Solanum* genus and using a suitable part or parts of the plant, in conj unction with appropriate operating conditions.

Preferably, the water-soluble extract is obtained from a plant of *Solanum* genus selected from the group consisting of *Solanum incanum* L. , *Solanum indicum, Solanum nigrum, Solanum capsicastrum*, *Solanum xanthocarpum, Solanum melongena, Solanum coagulans, Solanum tunigrum, Solanum sodomeum, Solanum turburosum, Solanum aculeastrum, Solanum lycocarpum, Solanum khasianum*, *Solanum suaveolens, Solanum uporo, Salanum abutiloides*, *Solanum coccineum, Solanum unguiculatum, Solanum robustum, Solanum anguivi, Solanum platanifolium,* and *Solanum mammosum.* In a preferred embodiment of this invention, the water-soluble extract is obtained from *Solanurn incanum* L..

In preliminary human tests done by the inventors, the results show that the water-soluble extract is effective in treating or alleviating warts including cutaneous warts and mucosal warts without undesired side effects, e.g., ulceration or erythema. Therefore, the present invention provides a method for treating wart, which comprises applying to a subject in need of such treatment the water-soluble extract.

The wart that can be treated by the anti-wart pharmaceutical composition of this invention includes common wart, flat wart, plantar wart, and genital wart.

The anti-wart pharmaceutical composition of this invention can be administered via e.g., parenteral, transmucosal, transdermal, or topical route.

The parenteral route includes subcutaneous injection, intraepidermal injection, intradermal injection, and intralesional injection.

For the parenteral route, the anti-wart pharmaceutical composition of this invention can be formulated into an injection product using techniques known to a skilled artisan, e. g. , sterile aqueous solution or dispersion.

In this invention, the injection product can be prepared by mixing the water-soluble extract with a pharmaceutically acceptable carrier that is widely employed in drug-manufacturing technology.

The pharmaceutically acceptable carrier comprises one or more reagents, including, for example, water, saline, buffer solution (Phosphate Buffered Saline (PBS), Ringer's solution or Hank's solution), emulsifier, suspending agent, decomposer, pH adjusting agents, stabilizing agent, chelating agent, preservative, diluents, absorption delaying agent, liposome, etc. The choice and amount of these pharmaceutically acceptable carriers are within the expertise of those skilled in the art.

Moreover, the anti-wart pharmaceutical composition of this invention can be administered by topical routes, e.g., dermal, buccal, genital, vaginal, anal, and rectal routes. In an embodiment of this invention, the anti-wart pharmaceutical composition of this invention is administered by dermal route. In another embodiment of this invention, the anti-wart pharmaceutical composition of this invention is administered by genital route.

The pharmaceutical composition according to this invention can be formulated into a suitable dosage form for topical administration using technology well known to those skilled in the art, which includes, but is not limited to, external preparations, effervescent tablets, suppositories, and the like.

In a preferred embodiment of this invention, the anti-wart pharmaceutical composition is formulated into an external preparation by admixing the water-soluble extract according to this invention with a base that is well known and commonly used in the art.

In this invention, the external preparation includes, but is not limited to, emulsion, gel, ointment, cream, patch, liniment, powder, aerosol, spray, lotion, serum, paste, foam, drop, suspension, and tincture. In an embodiment of this invention, the anti-wart pharmaceutical composition is in the form of gel.

For example, the base suitable for producing the external preparation according to this invention may include one or more of the following additives: water, alcohols, glycol, hydrocarbons (e. g. , petroleum jelly and white petrolatum), wax (e.g., paraffin and yellow wax), preserving agents, antioxidant, surfactants, absorption enhancers, stabilizing agents, gelling agents (e.g., carbopol^{®}97 4P, microcrystalline cellulose, and carboxymethylcellulose),active agents, humectants, odor absorbers, fragrances, pH adjusting agents, chelating agent, emulsifier, occlusive agents, emollients, thickeners, solubilizing agents, penetration enhancers, anti-irritants, colorants, and propellants. The choice and amount of these additives are within the expertise of those skilled in the art.

The dosage and the frequency of administration of the anti-wart pharmaceutical composition according to this invention may vary depending on the following factors: the severity of the disease to be treated, the route of administration, and the weight, age, physical condition and response of the subject to be treated. For instance, the daily dosage of the pharmaceutical composition according to this invention may be 10 to 20 mg/cm² of the lesion area and one to six times per day.

This invention will be further described by way of the following examples. However, it should be understood that the following examples are solely intended for the purpose of illustration and should not be construed as limiting the invention in practice.

### Example 1: Preparation of the water-soluble extract

The water-soluble extract was prepared based on the procedure as disclosed in Example 1 of US 7,078,063 B2. Specifically, 500 g of ripe fruit of *Solanum incanum* L. was ground subsequent to addition of 1000 ml pure water. To the resultant aqueous mixture, 99.5% of acetic acid was added dropwise to adjust the pH value to 4.0, followed by shaking at room temperature for 12 hrs. A supernatant was obtained by centrifuging the aqueous mixture at 4500 rpm for 12 hours, and 33% NH₄OH basic solution was added thereto dropwise to adjust the pH value of the supernatant to 9.0, and a precipitate was formed. The precipitate was obtained by conducting centrifugation at 4,500 rpm for 10 minutes(Beckman Coulter, Avanti J-25, JA-14 Rotor), and the residual basic solution present therein was removed by washing the precipitate with water, followed by centrifugation at 4,500 rpm.The precipitate thus obtained was suspended in distilled water and subjected to lyophilization (Virtis, Freezemobile 12ES) to obtain 5 g of dried powder.

2 g of the dried powder was dissolved in 50 ml chloroform in reagent grade, followed by addition of 40 ml of 100% methanol and shaking to form a uniform suspension. A supernatant was obtained by centrifugation at 4,500 rpm. 70 ml of methanol:water solution (1:1) was added to the supernatant and mixed well. The mixture obtained was centrifuged at 12,000 rpm for 10 min. The resultant supernatant was taken out, and 120 ml distilled water was added thereto and shaken well. The supernatant was further centrifuged at 12,000 rpm for 10 min so as to remove the precipitate. The resultant supernatant was subjected to concentration under reduced pressure at 55°C to remove methanol, followed by lyophilization to obtain dried powder of the water-soluble extract.

### Example 2: Therapeutic effect and safety of the water-soluble extract in treating human cutaneous wart and mucosal wart

In order to examine the therapeutic effect and safety of the water-soluble extract as prepared in the above Example 1 in treating human cutaneous wart and mucosal wart, the following tests were conducted.

### A. Preparation of gel containing the water-soluble extract

4 g of carbopol^{®}974P, which was used as a gelling agent and is commercially available from Lubrizol Advanced Materials, Inc. , KY 40258, USA, was dissolved in 50 g pure water, followed by sequential addition of 30 g of propylene glycol and 7 g of the dried powder of the water-soluble extract obtained in Example 1 and mixing well. The mixture was heated in a heating vessel at a temperature of 50°C to 60°C for 20 minutes, followed by cooling at room temperature. The cooled mixture was added with triethanolamine to adjust pH to 7.0±0.5. Subsequently, water was added until the total weight of the mixture reached 100 g, thereby obtaining a gel containing 7% (w/w) of the water-soluble extract (hereinafter referred to as *Solanum incanum* L. gel).

### B. Test subjects and clinical information thereof

Six test subjects (test subjects A to F) participating in the following tests were enrolled from the outpatient department of National Cheng Kung University Hospital, Taiwan. The test subjects A and B suffered from mucosal wart, and the test subjects C to F suffered from cutaneous wart. The clinical information of the six test subjects, including gender, age, wart type, and lesion location, are outlined in Table 1.

**Table 1**

| Test subject | Age (y/o) | Gender | Wart type | Lesion location |
|---|---|---|---|---|
| A | 32 | Female | Mucosal wart | External genital |
| B | 21 | Female | Mucosal wart | External genital |
| C | 28 | Female | Cutaneous wart | Finger tip |
| D | 21 | Male | Cutaneous wart | Finger tip, finger |
| E | 26 | Male | Cutaneous wart | Finger |
| F | 33 | Male | Cutaneous wart | Finger tip, palm |

### C. Pre-clinical test

Prior to the start of the test (i.e., on Week 0), selected lesions on each of the enrolled test subjects were photographed. The *Solanum incanum* L. gel prepared in section A of Example 2 was applied to the selected lesions and normal skin areas that surround the selected lesions. Each of the normal skin areas had inner and outer peripheries and the distance between the inner and outer peripheries of each of the normal skin areas was not greater than 0.5 cm. The treated dosage was 10∼20 mg/cm², and twice a day. The selected lesions of each of the test subjects were photographed at predetermined evaluation times (i.e., at the ends of weeks 1, 2, 3, 4, 7, 8, 13, 20 and 29, see Table 2), and evaluations for therapeutic effect and safety of the *Solanum incanum* L. gel were conducted according to the procedures set forth in sections D and E below. The test was finished when the lesions of each of the test subjects were completely cured and the outer appearance of the lesion skin was recovered to a normal state.

**Table 2**

| Test subject | Evaluation time (week) | | | | | | | | | |
|---|---|---|---|---|---|---|---|---|---|---|
| | 0^{a} | 1^{b} | 2 | 3 | 4 | 7 | 8 | 13 | 20 | 29 |
| A | +^{c} | + | -^{d} | - | - | - | - | - | - | - |
| B | + | + | - | - | - | - | - | - | - | - |
| C | + | - | - | - | + | - | + | - | - | - |
| D | + | - | + | + | - | - | + | - | - | - |
| E | + | - | + | - | - | + | - | - | - | - |
| F | + | - | - | - | - | + | - | ⊚^{e} | 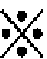 ^{f} | + |

| | | | | | | | | | | |
|---|---|---|---|---|---|---|---|---|---|---|
| ^{a} : "0" indicates week 0, i.e. , prior to application of the *Solanum incanum* L. gel ^{b} : "1" means week 1 after application of the *Solanum incanum* L. gel ^{c} : "+" means evaluation was conducted ^{d} : "-" means no evaluation was conducted ^{e} : "⊚" means evaluation was conducted only on the lesions at finger tip ^{f} : " 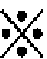 "means evaluation was conducted only on the lesion on palm | | | | | | | | | | |

### D. Evaluation of therapeutic effect

The photos of the selected lesions of the test subjects as obtained at Week 0 (i.e., prior to the application of the *Solanum incanum* L*.* gel) and as obtained at the evaluation times were compared by a dermatologist to determine the improvement of lesions treated with the *Solanum incanum* L. gel.

### E. Evaluation of safety

Evaluation of safety was conducted by a researcher at the predetermined evaluation times. The test subjects were interrogated by the researcher in respect to the appearance of any adverse skin response (including ulceration, irritation and erythema).

### Results:

### A. Evaluation of the therapeutic effects of the Solanum incanum L. gel

Compared with the photos of the selected lesions of each of the test subjects A and B as obtained at week 0 and other predetermined evaluation times, the selected lesions of each of the test subjects A and B treated with the *Salanum incanum* L. gel were significantly improved after the end of week 1 (data not shown).

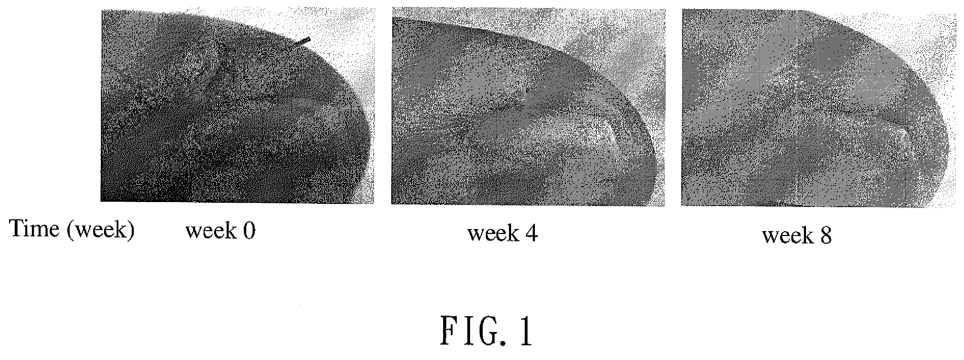

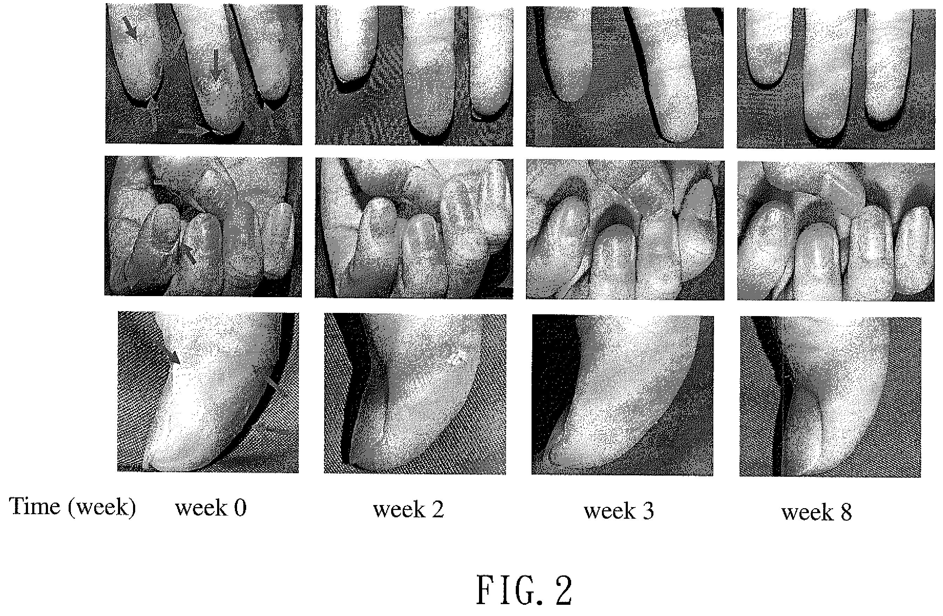

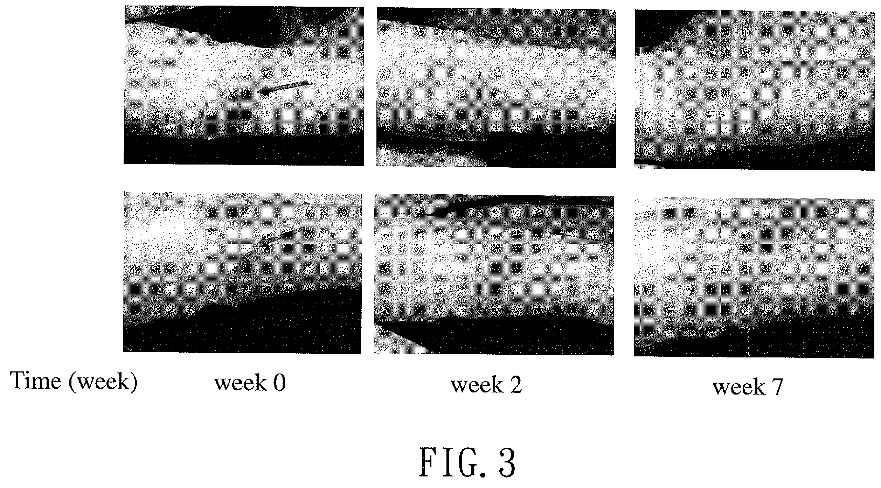

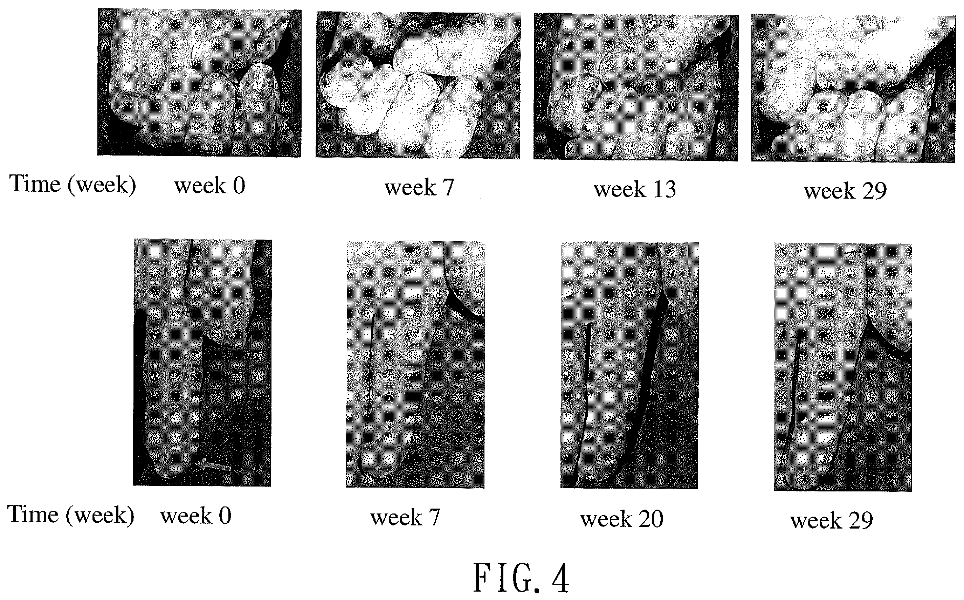

Figs. 1 to 4 respectively show photos of the selected lesions of the test subjects C to F as obtained at different evaluation times. The photos demonstrate that the lesions of each of the test subjects C to F treated with the *Solanum incanum* L. gel greatly improved with time. After the test was finished, the test subjects C to F were further sub j ected to the follow-up study for six months to two years to determine whether the lesion area that had already been cured by the *Solanum incanum* L. gel was recurrent. Based on the results of the follow-up study, no warts were found to have recurred for all of the test subjects (data not shown).

The aforesaid results show that the *Salanum incanum* L. gel of this invention could be effectively used to treat cutaneous wart and mucosal wart.

### B. Evaluation of the safety of the gel containing the water-soluble extract

In the safety test, only a few of the test subjects experienced temporary and mild adverse skin responses, including irritation or erythema, which, however, did not halt them from completing the test (data not shown).

In view of the aforesaid, the inventors contemplate that the water-soluble extract from a plant of *Solanum* genus according to this invention can be developed into an anti-wart drug for long-term use in treating warts without adverse side effects.

## Claims

1. A water-soluble extract from a plant of *Solanum* genus for use in the treatment of warts on the skin or mucous of a human or animal subject, said water-soluble extract comprising solamargine and solasonine, and said water-soluble extract being obtainable by the following steps:
(a) subjecting a plant material of a plant of *Solanum* genus to an extraction treatment using an acidic aqueous solution with a pH value of 3~5, such that an aqueous solution is obtained;
(b) adjusting the pH value of the aqueous solution obtained in step (a) to pH 8-10 with a base, such that a precipitate is formed;
(c) washing the precipitate formed in step (b) with water, followed by drying, such that a dried product is obtained;
(d) admixing the dried product obtained in step (c) with chloroform, followed by addition of a suitable amount of a 100% alcohol, such that a chloroform-alcohol mixture is formed;
(e) mixing the chloroform-alcohol mixture formed in step (d) with a water/alcohol solution having a predetermined water:alcohol ratio, such that a mixture containing a chloroform-based layer and a non-chloroform-based layer is obtained;
(f) removing the chloroform-based layer from the mixture obtained in step (e), followed by addition of a suitable amount of water; and
(g) obtaining a supernatant from the resultant mixture of step (f), followed by drying the supernatant, wherein the resultant dried product is able to be directly dissolved in water to form a yellowish clear and transparent aqueous solution.

2. An extract of claim 1 for a use as defined in claim 1, wherein said water-soluble extract comprises at least 60 wt% of solamargine and solasonine.

3. An extract of claim 1 for a use as defined in claim 1, wherein said water-soluble extract comprises 60 wt%-90 wt% of solamargine and solasonine.

4. An extract of claim 1, 2, or 3 for a use as defined in claim 1, wherein the extract is for use in treating common wart, flat wart, plantar wart, or genital wart on the subject.

5. An extract of claim 1, 2, 3, or 4 for a use as defined in claim 1, wherein the extract is applied in a topical dosage form.

6. An extract of claim 5 for a use as defined in claim 5, wherein the extract is further **characterized by** a base selected from the group consisting of water, alcohols, glycol, hydrocarbons, wax, preserving agents, antioxidant, surfactants, absorption enhancers, stabilizing agents, gelling agents, active agents, humectants, odor absorbers, fragrances, pH adjusting agents, chelating agent, emulsifier, occlusive agents, emollients, thickeners, solubilizing agents, penetration enhancers, anti-irritants, colorants, propellants, and combinations thereof.

7. An extract claim 6 for a use as defined in claim 5, wherein the extract is further **characterized in that** the base includes a gelling agent and glycol.

## Patentansprüche

1. Wasserlöslicher Extrakt aus einer Pflanze der Gattung *Solanum* zur Anwendung bei der Behandlung von Warzen auf der Haut oder Schleimhaut eines menschlichen oder tierischen Subjekts, wobei der wasserlösliche Extrakt Solamargin und Solasonin umfasst, und wobei der wasserlösliche Extrakt mithilfe der folgenden Schritte gewonnen werden kann:
(a) Unterziehen eines Pflanzenmaterials einer Pflanze der Gattung *Solanum* einer Extraktionsbehandlung unter Verwendung einer sauren wässrigen Lösung mit einem pH-Wert von 3 ∼ 5, sodass eine wässrige Lösung gewonnen wird;
(b) Einstellen des pH-Werts der in Schritt (a) gewonnenen wässrigen Lösung mit einer Base auf einen pH-Wert von 8 ∼ 10, sodass ein Präzipitat gebildet wird;
(c) Waschen des in Schritt (b) gebildeten Präzipitats mit Wasser, gefolgt von Trocknen, sodass ein getrocknetes Produkt gewonnen wird;
(d) Vermengen des in Schritt (c) gewonnenen getrockneten Produkts mit Chloroform, gefolgt von der Zugabe einer geeigneten Menge eines 100-prozentigen Alkohols; sodass ein Chloroform-Alkohol-Gemisch gebildet wird;
(e) Mischen des in Schritt (d) gebildeten Chloroform-Alkohol-Gemisches mit einer Wasser/Alkohol-Lösung in einem vorab festgelegten Wasser:Alkohol-Verhältnis, sodass ein Gemisch gewonnen wird, das eine auf Chloroform basierende Schicht und eine nicht auf Chloroform basierende Schicht enthält;
(f) Entfernen der auf Chloroform basierenden Schicht aus dem in Schritt (e) gewonnenen Gemisch, gefolgt von der Zugabe einer geeigneten Menge Wasser; und
(g) Gewinnen eines Überstands aus dem resultierenden Gemisch aus Schritt (f), gefolgt von Trocknen des Überstands, wobei das sich ergebende getrocknete Produkt direkt in Wasser gelöst werden kann, um eine gelbliche klare und transparente wässrige Lösung zu bilden.

2. Extrakt nach Anspruch 1 zur Anwendung nach Anspruch 1, wobei der wasserlösliche Extrakt mindestens 60 Gew.-% Solamargin und Solasonin umfasst.

3. Extrakt nach Anspruch 1 zur Anwendung nach Anspruch 1, wobei der wasserlösliche Extrakt 60 Gew.-% - 90 Gew.-% Solamargin und Solasonin umfasst.

4. Extrakt nach Anspruch 1, 2, oder 3 zur Anwendung nach Anspruch 1, wobei der Extrakt zur Anwendung bei der Behandlung von gewöhnlichen Warzen, Flachwarzen, Dornwarzen oder Genitalwarzen des Subjekts bestimmt ist.

5. Extrakt nach Anspruch 1, 2, 3, oder 4 zur Anwendung nach Anspruch 1, wobei der Extrakt in einer topischen Darreichungsform angewandt wird.

6. Extrakt nach Anspruch 5 zur Anwendung nach Anspruch 5, wobei der Extrakt des Weiteren durch eine Base gekennzeichnet ist, die aus der Gruppe ausgewählt ist, bestehend aus Wasser, Alkoholen, Glycol, Kohlenwasserstoffen, Wachs, Konservierungsmitteln, Antioxidationsmitteln, Tensiden, Absorptionsaktivatoren, Stabilisierungsmitteln, Geliermitteln, Wirkstoffen, Feuchthaltemittel, Geruchsabsorptionsmitteln, Duftstoffen, Mitteln zur Einstellung des pH-Werts, Chelatbildnern, Emulgatoren, okklusiven Mitteln, Weichmachern, Verdickungsmitteln, Lösungshilfsmitteln, Penetrationsförderern, reizmildernden Mitteln, Farbstoffen, Treibmitteln und Kombinationen davon.

7. Extrakt nach Anspruch 6 zur Anwendung nach Anspruch 5, wobei der Extrakt des Weiteren **dadurch gekennzeichnet ist, dass** die Base ein Geliermittel und Glycol beinhaltet.

## Revendications

1. Extrait hydrosoluble de plante du genre *Solarium* pour une utilisation dans le traitement des verrues sur la peau ou les muqueuses d'un sujet humain ou animal, ledit extrait hydrosoluble comprenant de la solamargine et de la solasonine et ledit extrait hydrosoluble pouvant être obtenu par les étapes suivantes consistant à :
(a) soumettre un matériel végétal d'une plante du genre *Solanum* à un traitement d'extraction à l'aide d'une solution aqueuse acide ayant une valeur de pH de 3~5, de sorte qu'une solution aqueuse est obtenue ;
(b) ajuster la valeur de pH de la solution aqueuse obtenue à l'étape (a) à un pH de 8~10 avec une base, de sorte qu'un précipité est formé ;
(c) laver le précipité formé à l'étape (b) avec de l'eau, puis le sécher, de sorte qu'un produit séché est obtenu ;
(d) mélanger le produit séché obtenu à l'étape (c) avec du chloroforme, puis ajouter une quantité appropriée d'un alcool à 100 %, de sorte qu'un mélange chloroforme-alcool est formé ;
(e) mélanger le mélange chloroforme-alcool formé à l'étape (d) avec une solution eau/alcool ayant un rapport eau:alcool prédéterminé, de sorte qu'un mélange contenant une couche à base chloroforme et une couche à base non-chloroforme est obtenu ;
(f) éliminer la couche à base chloroforme du mélange obtenu à l'étape (e), puis ajouter une quantité appropriée d'eau ; et
(g) obtenir un surnageant à partir du mélange résultant de l'étape (f), puis sécher le surnageant, le produit séché résultant étant en mesure d'être directement dissout dans de l'eau pour former une solution aqueuse jaunâtre claire et transparente.

2. Extrait selon la revendication 1 pour une utilisation telle que définie dans la revendication 1, où ledit extrait hydrosoluble comprend au moins 60 % en poids de solamargine et de solasonine.

3. Extrait selon la revendication 1 pour une utilisation telle que définie dans la revendication 1, où ledit extrait hydrosoluble comprend 60 % en poids à 90 % en poids de solamargine et de solasonine.

4. Extrait selon la revendication 1, 2 ou 3 pour une utilisation telle que définie dans la revendication 1, où l'extrait est destiné à être utilisé dans le traitement des verrues vulgaires, les verrues planes, les verrues plantaires ou les condylomes sur le sujet.

5. Extrait selon la revendication 1, 2, 3 ou 4 pour une utilisation telle que définie dans la revendication 1, où l'extrait est appliqué sous une forme galénique topique.

6. Extrait selon la revendication 5 pour une utilisation telle que définie dans la revendication 5, où l'extrait est en outre **caractérisé par** une base choisie dans le groupe constitué par l'eau, les alcools, un glycol, les hydrocarbures, la cire, les agents conservateurs, un antioxydant, les tensioactifs, les activateurs d'absorption, les agents stabilisants, les agents gélifiants, les agents actifs, les humectants, les absorbeurs d'odeur, les parfums, les agents d'ajustement du pH, un agent chélatant, un émulsifiant, les agents occlusifs, les émollients, les épaississants, les agents solubilisants, les activateurs de pénétration, les anti-irritants, les colorants, les propulseurs et leurs combinaisons.

7. Extrait selon la revendication 6 pour une utilisation telle que définie dans la revendication 5, où l'extrait est en outre **caractérisé en ce que** la base comprend un agent gélifiant et un glycol.
